# EUROPÄISCHE PATENTSCHRIFT

(11) **EP 0 981 351 B1**
(45) Veröffentlichungstag und Bekanntmachung des Hinweises auf die Patenterteilung: **07.11.2001**
(21) Anmeldenummer: 98924293.8
(22) Anmeldetag: 08.05.1998
(51) Int. Cl.: A61K 31/66

(54) **VERWENDUNG VON DIPHOSPHONSÄUREN ODER DEREN PHYSIOLOGISCH VERTRÄGLICHEN SALZEN ODER ESTERN ZUR PRÄVENTIVEN BEHANDLUNG VON SPÄTFOLGEN BEI HARNBLASENERWEITERUNG ODER HARNBLASENERSATZ**
USE OF DIPHOSPHONIC ACIDS OR THE PHYSIOLOGICALLY ACCEPTABLE SALTS OR ESTERS THEREOF IN THE PREVENTIVE TREATMENT OF AFTER-EFFECTS RESULTING FROM ENLARGEMENT OR REPLACEMENT OF THE BLADDER
UTILISATION D'ACIDES DIPHOSPHONIQUES OU DE LEURS SELS OU ESTERS PHYSIOLOGIQUEMENT TOLERABLES POUR LE TRAITEMENT PREVENTIF DES SEQUELLES TARDIVES EN CAS D'AGRANDISSEMENT VESICAL OU DE REMPLACEMENT VESICAL

(30) Priorität: 09.05.1997 DE 19719680
(43) Veröffentlichungstag der Anmeldung: 01.03.2000
(73) Patentinhaber: Roche Diagnostics GmbH, 68298 Mannheim (DE)
(72) Erfinder: BAUSS, Frieder, D-67141 Neuhofen (DE); BRKOVIC, Drasko, D-69181 Leimen (DE)
(74) Vertreter: Witte, Hubert, Dr.
(86) Internationale Anmeldenummer: EP9802712
(87) Internationale Veröffentlichungsnummer: WO9851314

(56) Entgegenhaltungen:
- EP-A- 0 531 253
- EP-A- 0 566 535
- WO-A-94/14455
- WO-A-96/19998
- WO-A-96/35407
- MUNDY ET AL.: "Calcium balance, growth and skeletal mineralisation in patients with cystoplasties" BRITISH JOURNAL OF UROLOGY, Bd. 69, 1992, Seiten 257-259, XP002076297 in der Anmeldung erwähnt
- DATABASE WPI Week 9608 Derwent Publications Ltd., London, GB; AN 96-074761 XP002076298 & JP 07 330 613 A (TEIJIN LTD.)

## Beschreibung

Die Erfindung betrifft die Verwendung von Diphosphonsäuren oder deren physiologisch verträglichen Salzen oder Estern zur Herstellung eines Arzneimittels zur präventiven Behandlung von Spätfolgen, die nach operativer Harnblasenerweiterung durch Augmentationsplastiken oder Harnblasenersatz durch Ersatzplastiken auftreten können und sich oftmals erst Jahre nach der Operation zeigen.

Aus unterschiedlichen Gründen kann es notwendig sein, die Harnblase zu erweitern (Blasenaugmentationsplastik) oder gar zu ersetzen (Blasenersatzplastik). Gegenwärtig wird dieser Eingriff in der Regel unter Verwendung von unterschiedlichen Darmsegmenten oder auch von Magengewebe als Erweiterungs- oder Ersatzplastik vorgenommen.

Die häufigste Indikation zur Erweiterungsplastik im Kindes- und Jugendalter sind Patienten mit funktionellen Blasenentleerungsstörungen (Spina bifida, Kinder mit offenenen oder geschlossenen Spaltbildungen), welche neben der Blasenentleerungsstörung oftmals auch eine Niereninsuffizienz unterschiedlichen Ausprägungsgrades aufweisen. Neuere Untersuchungen zeigen, daß Kinder mit Darminterponaten in der Harnblase oder mit vollständigem Ersatz der Harnblase durch Darm eine signifikante Verminderung des Längenwachstums um 20 bis 50% aufweisen (vgl. Mundy.A.R., Nurse D.E.: Calcium balance, growth and skeletall mineralisation in patients with cystoplasties. Br. J. Urol. 69; 257 - 259, 1992 und Wagstaff K.E., Woodhouse C.R.J., Duffy P.G., Ransley P.G.: Delayed linear growth in Children after enterocystoplsty. Br. J. Urol. 69; 314 - 317, 1992).

Wachstumsstörungen nach Blasenerweiterungsplastik sind im Kindes- und Jugendalter ein in den letzten Jahren vermehrt beobachtetes Phänomen, welches sowohl in der zugrunde liegenden Erkrankung als auch in der Wahl der zur Augmentation verwendeten Darmsegmente begründet ist.

Ossäre Mineralisationsstörungen lassen sich auch beim Erwachsenen mit uneingeschränkter Nierenfunktion nach Darmincerposition in die Blase nachweisen (Whitemore W.F. und Gittes R.F. (1983), J. Urol. 129, 494-498). Jedoch stellen Patienten ohne abgeschlossenes Längenwachstum und renaler Insuffizienz eine besondere Risikogruppe dar.

Als eine Ursache eines sicher vielschichtigen Problems könnte eine resorptive hyperchlorämische Azidose mit konsekutiven Schäden im Knochensystem eine Rolle spielen(Koch M.O. und W.S. McDougal (1985), Surgery 98, 561).

Um das Auftreten von Azidosen und deren Folgeschäden bei Harnblasenerweiterungs- oder -ersatzplastiken auszuschließen, wird zur Zeit postoperativ Natriumbicarbonat zur Neutralisierung verabreicht. Da Azidosen jedoch nur bei einem Bruchteil der Patienten nach einer Blasenerweiterungs- oder Blasenersatzplastik aufzufinden sind, ist die Alkali- Therapie für eine generelle Vermeidung der genannten Operationsspätfolgen unzureichend. Auch handelt es sich bei den Harnblasenerweiterungs- oder ersatzplastiken um ein relativ neues Harnableitungsverfahren, dessen Folgerisiken und - mechanismen noch nicht erschöpfend erforscht sind.

Der Erfindung lag die Aufgabe zugrunde, eine wirksame Therapie zur Verhinderung von Folgeschäden nach dem Einsetzen einer Blasenerweiterungs- oder Blasenersatzplastik vorzuschlagen, die sowohl für Erwachsene als auch für Kinder, sowohl für Patienten mit renaler Insuffizienz als auch für Patienten ohne Niereninsuffizienz anwendbar ist.
Es wurde überraschend festgestellt, daß die oftmals erst Jahre nach dem Einsetzen einer Augmentationsplastik oder einer Ersatzplastik auftretenden Spätfolgen auf das Knochensystem verhindert oder zumindest stark vermindert werden können, wenn den Patienten vor, während und/oder nach dem operativen Eingriff Diphosphonsäuren oder deren physiologisch verträglichen Salze oder Ester appliziert werden. Die Behandlung nach dem operativen Eingriff kann sofort oder nach Abklingen der Antibiotikagabe beginnen.

Gemäß der Erfindung wird mindestens eine Diphosphonsäure oder ein physiologisch verträgliches Salz davon verabreicht. Es ist auch möglich, mehrere Diphosphonsäuren bzw. deren physiologisch verträglichen Salze kombiniert zu verabreichen.

Bevorzugt handelt es sich bei der verwendeten Gruppe der Diphosphonate um Ibandronat, Etidronat, Clodronat, Risedronat, Pamidronat, Zoledronat, Incadronat, Tiludronat, Neridronat, Olpadronat, EB-1053 ([1-Hydroxy-3-(1-pyrrolidinyl)-propyliden]bis-phosphonat), YH 529 ([1-Hydroxy-2-imidazo-(1,2-a)pyridin-3-ylethyliden]bis-phosphonat) oder Alendronat. Prinzipiell können auch andere Diphosphonsäuren oder die physiologisch unbedenklichen Salze oder physiologisch unbedenklichen Ester der Diphosphonsäuren eingesetzt werden.

Besonders bevorzugt werden Ibandronsäure (1-Hydroxy-3-(N-methyl-N-pentyl)aminopropyliden-1,1-diphosphonsäure) oder deren physiologisch verträgliche Salze verwendet.

Diphosphonsäuren sind zur Behandlung von Kalziumstoffwechselerkrankungen bekannt. Sie enthaltende Arzneimittel werden zur Behandlung der Hyperkalzämie verwendet, aber auch zur Behandlung von Tumorosteolyse infolge Knochenmetastasen oder zur Behandlung von Osteoporose, siehe z.B. EP-A-0 566 535.

Die erfindungsgemäße Verwendung unmittelbar vor, während und/oder nach der Harnblasenaugmentation oder beim Harnblasenersatz zur Herstellung eines Arzneimittels zur präventiven Behandlung von Knochenstoffwechselstörungen lag für den operierenden Urologen nicht auf der Hand. Es handelt sich hier um eine neue Patientengruppe, die vorbeugend oder auch therapeutisch mit Diphosphonsäuren behandelt wird.

Die Diphosphonate können in flüssiger, fester oder in Form von Aerosolen oral, enteral, parenteral, topisch, transdermal, nasal, pulmonal oder rectal in allen üblichen nichttoxischen pharmazeutisch akzeptierten Trägermaterialien, Adjuvantien und Zusätzen verabreicht werden. Der Begriff parenteral umfaßt dabei subcutane, intravenöse und intramusculäre Zufuhr oder Infusionen. Orale Applikationsformen können z.B. Tabletten, Kapseln, Dragees, Sirupe, Lösungen, Suspensionen, Emulsionen, Elixiere etc. sein, die einen oder mehrere Zusätze aus den folgenden Gruppen enthalten können, wie z.B. Geschmacksstoffe, Süßstoffe, Farbstoffe und Konservierungsmittel. Orale Applikationsformen enthalten den wirksamen Bestandteil zusammen mit nichttoxischen, pharmazeutisch akzeptierten Trägermaterialien, die zur Herstellung von Tabletten, Kapseln, Dragees usw. geeignet sind, wie z.B. Calciumcarbonat, Natriumcarbonat, Lactose, Calciumphosphat oder Natriumphosphat; Stärke, Mannit, Methylcellulose, Talkum, hochdisperse Kieselsäuren, höhermolekulare Fettsäuren (wie Stearinsäure), Erdnußöl, Olivenöl, Paraffin, Miglyol, Gelatine, Agar-Agar, Magnesiumstearat, Bienenwachs, Cetylalkohol, Lecithin, Glycerol, tierische und pflanzliche Fette, feste hochmolekulare Polymere (wie Polyethyienglykole). Tabletten, Kapseln, Dragees usw. können mit einem entsprechenden Überzug, wie z.B. Glycerylmonostearat oder Glyceryldistearat versehen werden, so daß unerwünschte Nebenwirkungen im Magen verhindert werden, oder es durch die verzögerte Absorption im Gastrointestinaltrakt zu einer längeren Wirkungsdauer kommt. Als Injektionsmedium kommen vorzugsweise sterile injizierbare wäßrige oder ölige Lösungen oder Suspensionen zur Anwendung, welche die üblichen Zusätze, wie z. B. Stabilisierungsmittel und gegebenenfalls Lösungsvermittler enthalten. Derartige Zusätze können z.B. Wasser, isotonische Kochsalzlösung, 1,3-Butandiol, Fettsäuren (wie Ölsäure), Mono- und Diglyceride, oder Miglyol sein. Für die rectale Anwendung können alle geeigneten nicht irritierenden Zusätze verwendet werden, die bei normalen Temperaturen fest und bei Rectaltemperatur flüssig sind, wie z.B. Kakaobutter und Polyethylenglykol. Für die Anwendung als Aerosol kommen die pharmazeutisch üblichen Trägermedien zur Anwendung. Für den äußerlichen Gebrauch finden Cremes, Tinkturen, Gele, Lösungen oder Suspensionen usw. mit den pharmazeutisch üblichen Zusätzen Anwendung.

Für die erfindungsgemäße Verwendung liegt Ibandronat als Injektionslösung vor, die den Wirkstoff in einer Menge von 0,05 - 2000 mg enthält. Gemäß ihrer relativen Potenz im Vergleich mit Ibandronat ergeben sich die Wirkstoffgehalte für die Injektionslösungen der anderen erfindungsgemäßen Diphosphonate.

Je nach klinischem Bild und medizinischem Ziel (Prävention und/oder Behandlung) können die Diphosphonsäuren täglich oder auch zyklisch intermittierend appliziert werden.

Die tägliche i.v. äquivalente Dosierung liegt vorzugsweise bei 0,1 bis 100 µg/kg Ibandronat. Für alle anderen erfindungsgemäßen Diphosphonate wird die Dosierung entsprechend ihrer relativen Potenz zu Ibandronat bestimmt.

Bei Verwendung anderer Applikationsformen muß die Dosierung entsprechend der Bioverfügbarkeit der Applikationsform eingestellt werden.

Anschließend wird die Erfindung an Ausführungsbeispielen näher erläutert.

### Beispiel 1

Präventive Wirkung von Diphosphonaten vor auftretenden Osteopenien nach Augmentationsplastik

Jeweils 12 weibliche Sprague-Dawley Ratten (6-8 Wochen alt, ca. 80-100 kg Körpergewicht) wurden mit Magen- und Ileumaugmentationsplastiken versehen, und 12 Tiere wurden scheinoperiert. Daneben wurden 24 Tiere mittels ausgeschaltetem Sigma augmentiert und randomisiert in 2 Gruppen aufgeteilt, wobei die eine Gruppe eine tägliche s.c. Applikation von 0,9 % NaCl (Kontrolle) und die andere Gruppe eine tägliche s.c. Applikation von Ibandronsäure in Form einer wäßrigen Lösung in einer Dosis bis zu 20 µg P/kg erhielt. Die operative Gestaltung der Augmentationsplastiken erfolgte nach Lauvetz et al (J. Urol. 154: 899-902, 1995). Der Versuchszeitraum betrug 4 Monate, was ca. 4 Menschenjahren entspricht (Grimm E, Amer. J. Dig Dis. 7: 17-20, 1962).

Im Rahmen der monatlichen radiologischen Knochendichtemessungen (DEXA) zeigte sich im Vergleich zur Kontrollgruppe ein statistisch signifikantes Mineralisationsdefizit in den Lumbalwirbeln der Ileum-augmentierten Gruppe (p<0,01) und ein tendenzielles Defizit in der Magen- und Sigma-Gruppe ohne Ibandronat. In der Sigma-Gruppe, die mit Ibandronat behandelt wurde, zeigte sich keine reduzierte Knochenmasse.

### Beispiel 2

Zur Induktion einer chronischen Niereninsuffizienz (CNI) wurde bei 24 Ratten eine 5/6 Nephrektomie nach Kleinknecht et al (Contr. Nephrol.60: 27-38, 1988) vorgenommen; eine weitere Gruppe von 12 Tieren wurde scheinoperiert.
a) Bei den 12 scheinoperierten Tieren wird eine tägliche s.c. Applikation von 0,9 % NaCl vorgenommen.
b) Bei 12 Tieren mit CNI wird ebenfalls eine tägliche s.c. Applikation von 0,9 % NaCl vorgenommen.
c) Bei weiteren 12 Tieren mit CNI erfolgt eine tägliche s.c. Applikation von Ibandronsäure in Form einer wäßrigen Lösung in einer Dosis bis zu 20 µg P/kg.

Die Knochendichtemessung an den Lumbalwirbeln mittels DEXA erbrachte folgende Ergebnisse:

| Gruppe | a) NaCl | b) CNI + NaCl | c) CNI+Ibandronat |
|---|---|---|---|
| Reduktion der Knochenmasse | ± | +++ | ± |
| + = Grad der Knochenmassenreduktion gegenüber Kontrollen | | | |

### Beispiel 3

Zur Steigerung der klinischen Relevanz der gewonnenen Ergebnisse, wurde der Versuchsansatz von Beispiel 2 bei chronisch Nieren-insuffizienten Ratten wiederholt. Grundlage dieser dritten Versuchsanordnung ist die häufig anzutreffende Situation beim Menschen, in der eine vorgeschädigte Blase durch die vorgegebene Harntransportstörung (Reflux oder Obstruktion) sekundär zu einer Niereninsuffizienz führt. Es ist bekannt, daß die Niereninsuffizienz per se zur Knochenmineralisationsstörung (renale Osteopathie) führt, so daß die Notwendigkeit einer Blasenerweiterungsplastik mit Darmsegmenten das Risiko einer Mineralisationsstörung zusätzlich erhöht. Die Induktion der Niereninsuffizienz erfolgte durch 5/6 Nephrektomie (vgl. Beispiel 2). Die übrige Anordnung des Versuches entspricht Beispiel 1.

### Ergebnis:

Es zeigte sich, daß die osteopenische Wirkung der CNI durch die Augmentationen mittels der verwendeten Gastrointestinalsegmente deutlich verstärkt wurde. Die Verabreichung von Ibandronat verhinderte auch hier eine Reduktion der Knochenmasse.

## Patentansprüche

1. Verwendung von Diphosphonsäuren oder deren physiologisch verträglichen Salzen oder Estern zur Herstellung eines Arzneimittels zur präventiven Behandlung von ossären Mineralisationsstörungen bei Harnblasenerweiterung durch eine Augmentationsplastik oder Harnblasenersatz durch eine Ersatzplastik, zur Applikation vor, während und/oder nach dem operativen Einsetzen der Plastik.

2. Verwendung nach Anspruch 1, **dadurch gekennzeichnet, daß** eine Diphosphonsäure oder deren physiologisch verträgliches Salz oder eine Kombination von Diphosphonsäuren oder deren physiologisch verträglichen Salzen eingesetzt wird.

3. Verwendung nach den Ansprüchen 1 oder 2, **dadurch gekennzeichnet, daß** Ibandronat, Etidrcnat, Clodronat, Risedronat, Pamidronat, Zoledronat, Incadronat, Tiludronat, Neridronat, Olpadronat, EB-1053, YH 529 und/oder Alendronat eingesetzt werden.

4. Verwendung nach Anspruch 3, **dadurch gekennzeichnet, daß** Ibandronat oder dessen physiologisch verträgliche Salze oder Ester als Injektionslösungen mit einem Wirkstoffgehalt von 0,05 bis 2000 mg eingesetzt werden.

## Claims

1. Use of diphosphonic acids or physiologically compatible salts or esters thereof for the production of a medicament for the preventive treatment of disturbances to ossary mineralisation in the case of urinary bladder extension by an augmentation plasty or urinary bladder replacement by a replacement plasty for application before, during and/or after the operative use of the plasty.

2. Use according to claim 1, **characterized in that** a diphosphonic acid or physiologically compatible salt thereof or a combination of diphosphonic acids or their physiologically compatible salts is used.

3. Use according to claim 1 or 2, **characterized in that** ibandronate, etidronate, clodronate, risedronate, pamidronate, zoledronate, incandronate, tiludronate, neridronate, olpadronate, EB-1053, YH 529 and/or alendronate are/is used.

4. Use according to claim 3, **characterized in that** ibandronate or physiologically compatible salts or esters thereof is/are used as injection solutions with an active substance content of 0.05 to 2000 mg.

## Revendications

1. Utilisation d'acides diphosphoniques ou de leurs sels ou esters physiologiquement acceptables, pour la fabrication d'un médicament destiné au traitement préventif de troubles de la minéralisation osseuse dans l'élargissement de la vessie par une chirurgie plastique d'augmentation ou un remplacement de la vessie par une chirurgie plastique de remplacement, pour administration avant, pendant et/ou après la pose chirurgicale de la prothèse.

2. Utilisation selon la revendication 1, **caractérisée en ce qu'**on utilise un acide diphosphonique ou un sel physiologiquement acceptable de celui-ci, ou une association d'acides diphosphoniques ou de leurs sels physiologiquement acceptables.

3. Utilisation selon la revendication 1 ou 2, **caractérisée en ce qu'**on utilise l'ibandronate, l'étidronate, le clodronate, le risédronate, le pamidronate, le zolédronate, l'incadronate, le tiludronate, le néridronate, l'olpadronate, EB-1053, YH-529 et/ou l'alendronate.

4. Utilisation selon la revendication 3, **caractérisé en ce qu'**on utilise l'ibandronate ou ses sels ou esters physiologiquement acceptables, sous forme de solutions injectables ayant une teneur en substance active de 0,05 à 2 000 mg.
